# FASCICULE DE BREVET EUROPEEN

(11) **EP 1 753 391 B1**
(45) Date de publication et mention de la délivrance du brevet: **20.04.2011**
(21) Numéro de dépôt: 05773029.3
(22) Date de dépôt: 27.05.2005
(51) Int. Cl.: A61K 8/49, A61Q 19/06

(54) **UTILISATION D'ALKYLE FURANNES POUR LE TRAITEMENT COSMETIQUE DE LA CELLULITE**
VERWENDUNG VON FURANALKYLEN ZUR KOSMETISCHEN BEHANDLUNG VON ZELLULITIS
USE OF FURAN ALKYLS FOR A CELLULITE COSMETIC TREATMENT

(30) Priorité: 28.05.2004 FR 0405782; 21.03.2005 FR 0502756
(43) Date de publication de la demande: 21.02.2007
(73) Titulaire: Laboratoires Expanscience, 92400 Courbevoie (FR)
(72) Inventeur: PICCIRILLI, Antoine, 78670 Villennes sur Seine (FR); MSIKA, Philippe, F-78000 Versailles (FR); PICCARDI, Nathalie, F-21310 Arceau (FR)
(74) Mandataire: Ahner, Francis
(86) Numéro de dépôt international: PCT/FR2005/001311
(87) Numéro de publication internationale: WO 2005/117827

(56) Documents cités:
- EP-A- 0 110 548
- EP-A- 0 775 480
- EP-A- 1 020 192
- WO-A-01/21605
- WO-A-2004/002435
- WO-A-2004/112741

## Description

La présente invention concerne l'utilisation d'un ou plusieurs alkyles furannes, synthétiques ou naturels, dans le traitement consmétique.

Le diabète est une maladie chronique, souvent liée avec d'autres maladies comme la surcharge pondérale ou une dyslipidémie ou à un risque de maladies cardiovasculaires. Le diabète est aussi une cause principale de la cécité chez les personnes âgées de 25 à 74 ans. Les personnes souffrant du diabète sont 2 à 4 fois plus susceptibles de développer des maladies cardiaques et elles sont 5 fois plus susceptibles d'être victimes d'un accident vasculaire cérébral. Le diabète est défini comme un état d'hyperglycémie chronique : une glycémie à jeun supérieure à 7 mmol/l ou 1,26 g/l affirme le diabète. Si la glycémie est comprise entre 1,1 et 1,26 g/l, une hyperglycémie provoquée par voie orale doit être faite. On distingue deux types principaux de diabètes, le diabète insulino-dépendant et le diabète non-insulino-dépendant. Il existe aussi une autre forme de diabète qui touche certaines femmes enceintes.

Le diabète de type 1 ou insulino-dépendant est aussi appelé diabète juvénile car il se déclare le plus souvent dès l'enfance avec un pic de fréquence à 12 ans. Il concerne 10 à 15% des diabétiques. Cette forme de diabète débute brusquement et progresse rapidement. Il est considéré comme une maladie auto-immune, c'est-à-dire provoquée par le dérèglement du système immunitaire qui modifie ou détruit des cellules appartenant à l'organisme. Le système immunitaire détruit progressivement les cellules β des îlots de Langerhans du pancréas qui libère donc de moins en moins d'insuline. Quand toutes les cellules β sont détruites, il n'y a plus d'insuline produite. Cela signifie que le glucose, bien que disponible en quantité suffisante, ne peut être transporté dans les cellules pour la production énergétique. Pour contrebalancer cette déficience, il est vital d'injecter de l'insuline pour éviter une hausse de la glycémie. L'insuline ne peut pas être prise par voie orale, car elle est rendue inactive par l'estomac. La cause exacte de ce type de diabète n'est pas connue.

Le diabète de type II ou non-insulino-dépendant est le plus fréquent (près de 90 % des cas), cette forme est moins grave que la précédente et débute progressivement. Il débute généralement à l'âge adulte, après 40 ans, et est souvent lié à un surpoids. Chez la plus part des diabétiques de type 2, le pancréas produit suffisamment, voire trop d'insuline. Dans ce cas, l'insuline est bien produite par le pancréas mais insuffisamment ou en retard par rapport aux besoins ou bien le corps réagit en résistant à l'insuline Bien que l'organisme produise suffisamment d'insuline, les cellules ne peuvent pas réagir normalement, car les sites de reconnaissance situés à la surface de la cellule sont modifiés. L'insuline n'est plus reconnue et le glucose ne peut plus entrer en quantité suffisante dans les cellules pour produire de l'énergie. Trop de glucose reste dans le sang, il s'ensuit une glycémie élevée. Le pancréas, qui est responsable de la régulation du métabolisme du glucose, s'en rend compte et en conséquence produit davantage d'insuline. C'est le début d'un cercle vicieux: le pancréas produit de plus en plus d'insuline afin d'amener quand même du glucose aux cellules. C'est pourquoi, le diabétique de type 2 présente d'abord un taux d'insuline élevé. Les sites de reconnaissances doivent être modifiés de telle manière que l'insuline puisse être reconnue. La plupart des diabétiques de type 2 peuvent y parvenir par une perte de poids et une activité physique plus intense. Dans les cas plus avancés un traitement par comprimés se révèle nécessaire. Ces individus présentent un risque accru de développer une maladie cardiaque et de l'hypertension.

Il existe encore un autre type de diabète, le diabète gestationnel. Le terme de diabète gestationnel ou GDM (Gestational Diabetes Mellitus) est réservé à toute femme enceinte dont l'hyperglycémie ou l'intolérance au glucose se développe ou est diagnostiqué pour la première fois pendant la grossesse et disparaît à la fin du post-partum. Une femme ayant un diabète connu et devenant enceinte n'entre pas dans cette catégorie. Le diabète gestationnel est fréquent et concerne 6 % des grossesses. Il expose la mère et le foetus à de nombreux risques, tels qu'une augmentation de la fréquence de l'hypertension gravidique, de la pré-éclampsie et des césariennes, d'autant plus importants qu'il survient tôt et que l'hyperglycémie est mal maîtrisée. Chez l'enfant, il augmente le risque de mort néo-natale, de macrosomie, de traumatisme obstétrical, de détresse respiratoire, d'hypoglycémies néo-natales, d'hyperbilirubinémie et d'hypocalcémie. Outre les complications à court terme, les femmes ayant souffert d'un diabète gestationnel ont, 10 à 15 ans plus tard, un risque accru de développer un diabète de type II. Leurs enfants ont un risque d'obésité et, vraisemblablement, de diabète à long terme. Des études ont montré que les taux de magnésium dans le sérum et le liquide amniotique de mères diabétiques sont anormalement bas. Cette hypomagnésémie se répercute sur le foetus, pouvant provoquer des malformations congénitales ainsi que des hypocalcémies néo-natales précoces. Le traitement comporte un régime puis si la glycémie n'est pas normalisée et dépasse 7,8 mmol/l après un repas, l'administration d'insuline devient nécessaire.

De plus le diabète a des conséquences importantes sur la peau, en particulier le diabète de type 2. Il est connu que les sucres se lient aux protéines par réaction de Maillard entraînant une glycation des protéines (AGE) qui perdent leurs fonctionnalités et accroissent le vieillissement de la peau et des tissus globalement. Récemment , il a été montré que les lipides jouaient un rôle d'initiation de cette réaction irréversible et que des facteurs génétiques contribuaient à la sensibilité individuelle. Les taux les plus importants d'AGE se retrouvent dans les tissus à faible renouvellement comme les tendons, les os, la peau, le cartilage et les tissus amyloïdes. Ces AGE affectent les propriétés mécaniques de la matrice (baisse de l'élasticité) et limite le renouvellement des tissus. D'autres part, il a été mis en évidence une augmentation des métalloprotéases matricielles (MMP-2/3) et de troubles graves de la microcirculation qui sont des éléments essentiels dans la genèse de l'ulcère diabétique du pied qui est une pathologie provenant donc d'un défaut de cicatrisation sur un tableau inflammatoire chronique. Récemment, il a été mis en exergue une réduction de l'eau contenue dans la barrière cutanée des diabétiques ainsi qu'une diminution de la prolifération cellulaire épidermique et une modulation de la desquamation, traduisant ainsi un trouble important de la protection de la barrière cutanée similaire à la peau âgée, irritée ou xérotique. Le diabète limite également la capacité à réagir contre les infections bactériennes et fongiques ce qui peut transformer des blessures mineures en infection grave pouvant aller jusqu'à l'amputation d'un pied si des soins ne sont pas prescrits.

Une des principales causes du diabète non-insulino-dépendant (diabète de type 2) est l'obésité sans toutefois que celle ci soit invariablement liée au diabète. Les cellules adipeuses augmentent les besoins en insuline et les personnes obèses développent une résistance à son action. Le taux de glycémie ou de sucre dans le sang est alors constamment élevé, ce qui provoque le diabète. L'obésité peut également aggraver le diabète insulino-dépendant (diabète de type 1). On estime qu'une personne obèse a deux fois plus de risques de souffrir de diabète que les personnes dont le poids est normal. Des travaux récents, réalisés par une équipe de l'université de Laval, ont mis en évidence que des souris dont le gène iNOS (enzyme inductible de production du monoxyde d'azote) avait été génétiquement supprimé étaient protégées contre le développement du diabète associé à l'obésité (Nat Med. 2001 Oct; 7(10):1138-43.Targeted disruption of inducible nitric oxide synthase protects against obesity-linked insulin résistance in muscle. Perreault M, Marette A).

Outre son lien avec le diabète de type II et le diabète gestationnel, l'obésité est aujourd'hui le deuxième facteur après le vieillissement dans l'étiologie des pathologies chroniques. En 2004, environ 64% de la population adulte américaine présente un excédent de poids. On estime, qu'en 2008, 34% des américains adultes seront obèses. L'obésité est un excès de masse grasse dans le corps, résultat d'un déséquilibre entre l'apport calorique quotidien et les dépenses énergétiques : l'organisme reçoit plus qu'il ne dépense et donc "stocke" une partie du surplus. Cependant, de nombreux facteurs peuvent renforcer ce déséquilibre, et favoriser l'obésité ou du moins la prise de poids : l'hérédité, la culture alimentaire, la sédentarité, l'arrêt du tabac, des phénomènes hormonaux (notamment chez les jeunes et les femmes), la prise de médicaments, et le milieu socio-professionel... En moins de 3 ans, 650 000 nouveaux cas d'obésité ont été recensés en France. En Europe, et selon les pays, l'augmentation du nombre de personnes obèses varie entre 10 et 40 %. Ce taux record de 40 % est atteint chez les femmes des Pays de l'Est de l'Europe, en Tunisie où 40 % des personnes de plus de 30 ans présentent une obésité. Phénomène préoccupant, l'obésité infantile grimpe en flèche. Une étude en France sur des enfants de 10 mois à 8 ans a montré que 10 % d'entre eux étaient obèses au lieu des 3 % prévisibles. Au japon, l'obésité chez l'enfant a fait un bond de 53 %, en Angleterre de 65 % pour certaines tranches d'âges, et au USA de 60%.

Cette évolution inquiétante est source de nombreuses complications avec des conséquences endocriniennes, cardiovasculaires et psychosociales (discrimination, altération de la qualité de vie, douleur) qui demandent une prise en charge et donc un diagnostic aussi précoce que possible. Par exemple l'excès de poids augmente le risque de surmortalité cardiovasculaire (50 à80%), de maladies associées à une morbidité et une mortalité élevée (diabète de type 2 (possibilité de baisse de 30 à 45%), hypertension artérielle, troubles métaboliques, articulaires, vésiculaires) et de certains cancers (côlon, rectum, prostate, utérus, sein et vésicule). Il existe donc de nos jours un besoin important en médicaments adaptés pour le traitement du diabète et/ou de l'obésité.

L'amincissement passe préférentiellement par la lutte contre la surcharge pondérale localisée. Cette surcharge pondérale localisée se matérialise sous forme de graisses, dont la quantité et la répartition diffèrent en fonction du sexe. Ainsi, le tissu adipeux représente 20 à 30% du poids corporel chez la femme et 10 à 15% chez l'homme. La graisse sous-cutanée est deux fois plus épaisse chez la femme que chez l'homme. Chez l'homme les graisses s'accumulent autour et au-dessus de la ceinture (répartition androïde facteur de risque métabolique) et au-dessous de la ceinture, dans la région glutéo-fémorale chez la femme (répartition gynoïde, non corrélée à un risque vasculaire). L'une des caractéristiques de cette graisse accumulée en bas du corps est d'être difficilement mobilisable. Elle est destinée à assurer les besoins énergétiques de la reproduction (grossesse et, surtout, allaitement) et constitue ainsi le plus important réservoir énergétique de l'organisme.

Au niveau cellulaire, les adipocytes sont des cellules sphériques dont l'espace intracellulaire est comblé par une large vacuole remplie de triglycérides. Les adipocytes peuvent changer rapidement de volume. En effet, ces cellules peuvent atteindre, selon les circonstances 40 µm à 120 µm de diamètre, ce qui correspond à une augmentation de 27 fois en volume. Dans certains cas extrêmes, cette augmentation peut aller jusqu'à 40 fois. Ainsi, l'adipocyte est le principal acteur énergétique de l'organisme puisqu'il est capable de stocker (captation ou lipogenèse) ou, inversement, de mobiliser (lipolyse) rapidement les triglycérides, sources énergétiques majeures de l'organisme. La lipogenèse passe par la synthèse des triacylglycérols qui résulte de l'estérification du glycérol-3-phosphate par les acides gras activés ; à l'inverse, la lipolyse correspond à l'hydrolyse des triacylglycérols stockés, en glycérol et en acides gras. Différents mécanismes ont été mis en lumière, qui contrôlent la lipolyse et la lipogenèse qui font par exemple intervenir des récepteurs tels que les récepteurs alpha-2 et/ou béta-1 et -2, les récepteurs de l'adénosine de type A1, de la prostaglandine E2, Y2 de type YY et du neuropeptide NPY, mais aussi les hormones sexuelles.

Ainsi, la connaissance des mécanismes de contrôle de la lipolyse et de la lipogenèse adipocytaires s'est très nettement améliorée. Toutefois, des actifs amincissants sont toujours recherchés car les actifs amincissants connus ne sont pas totalement satisfaisants. Il existe donc à ce jour une réelle demande pour l'élaboration de compositions topiques permettant de favoriser efficacement l'amincissement.

La cellulite, ou lipodystrophie localisée, est caractérisée par une infiltration oedémateuse du tissu adipeux qui altère l'esthétique et l'harmonie de la silhouette. Si, pour des raisons diverses, telles qu'une nourriture trop riche, l'inactivité et/ou le vieillissement, un déséquilibre substantiel s'installe dans l'organisme entre la lipogenèse et la lipolyse, c'est à dire plus précisément si les quantités de graisses formées par lipogenèse deviennent notablement et constamment supérieures à celles qui sont éliminées par lipolyse, il se produit alors dans les adipocytes une accumulation de triglycérides, qui, si elle devient excessive, peut se traduire par une surcharge pondérale localisée et/ou progressivement par l'apparition d'une peau épaisse, à surface souvent irrégulière, d'aspect dit "peau d'orange", et de consistance plus ou moins flasque ou gélatineuse, donnant finalement à la silhouette un aspect général disgracieux. Ce tissu cellulitique n'épargne pas les hommes mais est nettement plus fréquent chez les femmes, qu'elles soient minces ou rondes. Les masses graisseuses se localisent préférentiellement sur la moitié inférieure du corps, au niveau des hanches, des cuisses, du ventre, sans oublier les genoux et les chevilles. La cellulite résulte notamment d'un stockage des triglycérides dans les adipocytes et d'une augmentation de la viscosité de la substance fondamentale du derme, qui se traduit par une rétention d'eau et une diminution des échanges cellulaires. Ces deux mécanismes entraînent une compression des vaisseaux sanguins et lymphatiques et une congestion des tissus.

D'une manière surprenante, les inventeurs ont découvert que les alkyles furannes, synthétiques ou naturels, pouvaient être utilisés dans le traitement du diabète et de l'obésité. Les inventeurs ont également découvert que l'application d'une composition topique comprenant ces alkyles furannes a une action amincissante et permet notamment de lutter contre la surcharge pondérale localisée et/ou la cellulite.

L'heptadécadienyl furane a déjà été décrit dans les demandes internationales WO 2004/112741 et WO 2004/002435 en tant qu'actif anti-âge. Cet actif anti-âge a été introduit dans les compositions amincissantes et/ou anti-cellulite décrites dans ces demandes internationales.

La présente invention décrit donc l'utilisation d'un ou plusieurs alkyles furannes, synthétiques ou naturels, pour la préparation d'un médicament destiné à la 5 prévention et/ou au traitement du diabète, notamment du diabète de type 2.

Dans le cadre de la présente description, on entend par l'emploi du terme « médicament » aussi bien les substances ou compositions présentant des propriétés curatives ou préventives à l'égard des maladies chez l'Homme que les compositions vétérinaires, à but prophylactique ou curatif.

Les alkyles furannes selon l'invention sont capables de diminuer le glucose sanguin (glycémie) et d'augmenter la tolérance au glucose. Ils peuvent ainsi être utilisé pour la fabrication d'un médicament ou de complément alimentaire ou dans l'alimentation courante pour la prévention et/ou le traitement du diabète et destiné à réguler la lipémie et/ou la glycémie et/ou la sensibilité à l'insuline.

La présente invention décrit également l'utilisation d'un ou plusieurs alkyles furannes, synthétiques ou naturels, pour la préparation d'un médicament destiné à réguler la lipémie et/ou la glycémie et/ou la sensibilité à l'insuline. Le médicament est aussi destiné à diminuer la masse graisseuse corporelle.

La lipémie correspond à la teneur du sang en lipides totaux circulants sous forme de lipoprotéines et d'acides gras libres. La présence dans le sang, de cette variété de lipides, également appelés triglycérides, peut être un signe de diabète. Les alkyles furannes selon l'invention sont capables de diminuer le taux de triglycérides circulants.

La glycémie correspond à la concentration de glucose mesurée dans le sang. Au-dessous d'un certain seuil, il y a hypoglycémie. Au-dessus d'un certain seuil, il y a hyperglycémie. La glycémie de référence est habituellement celle qu'on mesure à jeun le matin. Cependant, il peut être utile de mesurer d'autres glycémies préprandiales, c'est-à-dire le midi et au souper, ou des glycémies postprandiales, c'est-à-dire des glycémies mesurées après les repas (normalement 1 h 30 après), qui donnent des indications sur les glycémies les plus hautes dans la journée.

Les alkyles furannes selon l'invention sont capables de diminuer le taux de triglycérides dans le foie et les muscles, ils permettent ainsi de limiter le stockage des graisses. Les alkyles furannes selon l'invention augmente également le taux sanguin de

HDL (high density lipoprotein = lipoprotéine de haute densité), c'est-à-dire le bon cholestérol.

Les alkyles furannes selon l'invention permettent également d'augmenter les dépenses énergétiques (augmentation de PGC-1 dans les muscles - cf exemple 9).

La présente invention décrit également l'utilisation d'un ou plusieurs alkyles furannes, synthétiques ou naturels, pour la préparation d'un médicament destiné à la prévention et/ou au traitement de l'obésité.

Selon une variante avantageuse de l'invention, lesdits, alkyles furannes répondent à la formule générale suivante : 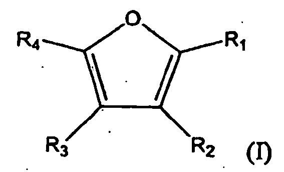 dans laquelle R₁, R₂, R₃ et R₄, identiques ou différents, représentent, indépendamment l'un de l'autre, un atome d'hydrogène, un radical alkyle en C₁-C₃₅, avantageusement en C₁₀-C₂₂, encore plus avantageusement en C₁₂-C₂₀, encore plus avantageusement en C₁₃-C₁₇, un radical alcényle en C₁-C₃₅, avantageusement en C₁₀-C₂₂, encore plus avantageusement en C₁₂-C₂₀, encore plus avantageusement en C₁₃-C₁₇ ou un radical alcynyle en C₁-C₃₅, avantageusement en C₁₀-C₂₂, encore plus avantageusement en C₁₂-C₂₀, encore plus avantageusement en C₁₃ - C₁₇, lesdits radicaux alkyles, alcényles et alcynyles pouvant être substitués par un ou plusieurs halogène(s) et/ou par une ou plusieurs fonctions choisies dans le groupe constitué par les fonctions époxyde, hydroxyle (-OH), thiol (-SH), éther (-OR₅), amine primaire (-NH₂), amine secondaire (-NHR₅), amine tertiaire (-NR₅R₆), aldéhyde (-CHO), cétone (-COR₅), acétyle (-O-CO-R₅), avec R₅ et R₆ représentant, indépendamment l'un de l'autre, un atome d'hydrogène, un radical alkyle en C₁-C₃₅, avantageusement en C₁₀-C₂₂, encore plus avantageusement en C₁₂-C₂₀, encore plus avantageusement en C₁₃-C₁₇ ou un radical alcényle en C₁-C₃₅, avantageusement en C₁₀-C₂₂, encore plus avantageusement en C₁₂-C₂₀, encore plus avantageusement en C₁₃-C₁₇.

Dans le cadre de la présente invention, les alkyles furannes préférés sont ceux qui sont monosubstitués en position 2. Ainsi, lesdits alkyles furannes sont avantageusement des 2-alkyle furannes synthétiques ou naturels répondant à la formule générale (I) dans laquelle R₁, R₂, R₃ représentent un atome d'hydrogène et R₄ ne représente pas un radical tel que défini précédemment autre que l'atome d'hydrogène.

Selon une variante avantageuse de l'invention, lesdits alkyles furannes sont des 2-alkyles furannes naturels notamment présents dans l'insaponifiable d'avocat furanique, répondant à la formule générale (I) dans laquelle R₁, R₂, R₃ représentent un atome d'hydrogène et R₄ représente un radical choisi dans le groupe constitué par les radicaux suivants (*-R₄) :

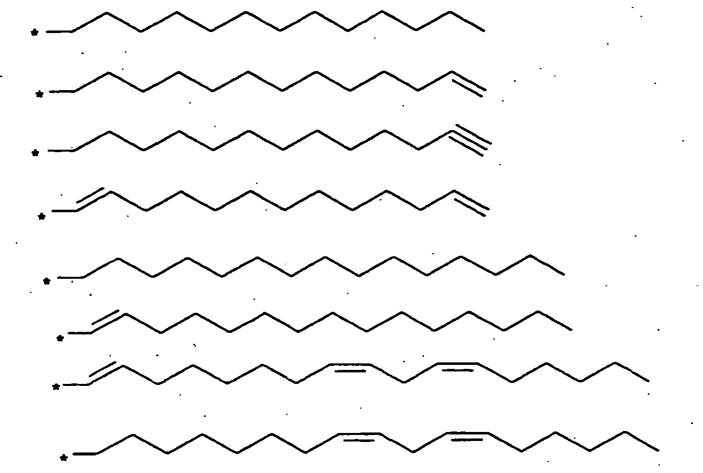

Dans l'insaponifiable d'avocat furanique, les 2-alkyles furannes représentent 30% à 70% en poids de cet insaponifiable, par rapport au poids total de l'insaponifiable. L'huile d'avocat peut contenir 2 à 4 % en poids de 2-alkyles furanes, par rapport au poids total de l'huile.

Selon une variante avantageuse de l'invention, on utilise une fraction furanique purifiée de l'insaponifiable d'avocat, comprenant 70 à 100% en poids, avantageusement 90 % à 100% en poids, de 2-alkyles furannes par rapport au poids total de la fraction,

L'insaponifiable est la fraction d'un corps gras qui, après action prolongée d'une base alcaline, reste insoluble dans l'eau et peut être extraite par un solvant organique. Cinq grands groupes de substances sont présents dans la plupart des insaponifiables d'huiles végétales : hydrocarbures saturés ou insaturés, alcools aliphatiques- ou terpéniques, stérols, tocophérols, les pigments caroténoïdes et xanthophiles. Les dérivés furaniques de l'huile d'avocat sont des composés connus de l'homme du métier, ils ont par exemple été décrits dans Farines, M. et al, 1995, J. of Am. Oil Chem. Soc. 72,473. L'insaponifiable d'avocat riche en lipides furaniques a déjà été décrit pour son utilisation dans la fabrication d'un médicament ayant une action bénéfique et curative sur le tissu conjonctif, notamment dans le traitement de pathologies inflammatoires telles que l'arthrose, les parodontites et la sclérodermie. Dans le cadre de la présente invention, pour l'insaponifiable d'avocat furanique, les expressions «alkyle furanne(s) » et « lipide(s) furanique(s) » sont des expressions synonymes:

L'avocat est avantageusement choisis parmi les variétés Hass, Fuerte, Ettinger, Bacon, Nabal, Anaheim, Lula, Reed, Zutano, Queen, Criola Selva, Mexicana Canta, légion Dschang, Hall, Booth, Peterson, Collinson Red, plus avantageusement, les variétés Hass, Fuerte et Reed. De préférence, on retiendra les variétés Hass, Fuerte, Ettinger et Bacon, et plus avantageusement les variétés Hass et Fuerte.

Dans la fraction furanique purifiée de l'insaponifiable d'avocat, telle que développée par les Laboratoires Expanscience (cf. la demande internationale WO 01/21605), les 2-alkyles furannes représentent de 70 à 100 % en poids, par rapport au poids de la fraction, et les proportions relatives massiques en chacun des furannes identifiés sont données dans le tableau 1 suivant :

**Tableau 1**

| *Composition en 2-alkylesfurannes de la fraction furanique purifiée de l'insaponifiable d'avocat* | *% en poids* |
|---|---|
| 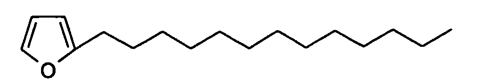 | 3-12 |
| 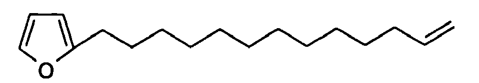 | 1-8 |
| 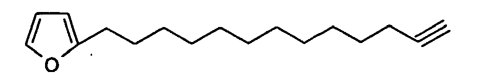 | 1-5 |
| 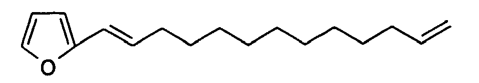 | 1-6 |
| 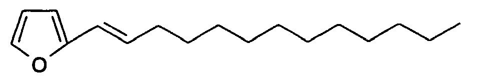 | 5-20 |
| 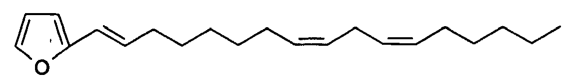 | 10-30 |
| 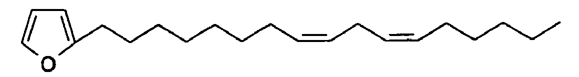 | 35-75 |

Dans la demande internationale WO 01/21605, les laboratoires Expanscience ont mis en oeuvre un procédé spécifique qui permet d'obtenir une extraction sélective des lipides furaniques de l'avocat avec une teneur de plus de 80% en poids de lipides furaniques, voire proche de 100%. Ce procédé comprend les étapes consistant à préparer un insaponifiable d'avocat, puis à soumettre l'insaponifiable d'avocat à une étape de distillation moléculaire en utilisant des moyens de température et de pression réglés pour obtenir un distillat comprenant principalement des lipides furaniques d'avocat.

De préférence, l'insaponifiable d'avocat est préparé à partir du fruit préalablement traité thermiquement, avant l'extraction de l'huile et la saponification, comme décrit en particulier dans la demande de brevet FR-91 08301. Ce traitement thermique consiste en un séchage contrôlé du fruit, frais de préférence, pendant au moins quatre heures, avantageusement entre 24 et 48 heures, à une température de préférence d'au moins environ 80°C et de préférence comprise entre environ 80 et environ 120°C ; la température et le temps de séchage étant dépendants l'un de l'autre. Avant sa saponification, l'huile peut être préalablement enrichie en insaponifiable en séparant une majorité des constituants de l'insaponifiable que l'on récupère dans un concentrat. Différentes méthodes peuvent être utilisées : cristallisation par le froid, extraction liquide-liquide, distillation moléculaire. La distillation moléculaire est particulièrement préférée et elle est réalisée avantageusement à une température comprise entre environ 180 et environ 230°C en maintenant une pression comprise entre 10⁻³ et 10⁻² mmHg. L'insaponifiable d'avocat obtenu comme décrit ci-dessus est ensuite soumis à une étape de distillation moléculaire. Cette étape de distillation moléculaire est réalisée avec des moyens de température réglés pour une température comprise entre 100 et 160°C et des moyens de pression réglés pour une pression comprise entre 10⁻³ et 5.10⁻² mmHg. En particulier, les moyens de température sont réglés pour une température comprise entre 100 et 140°C et les moyens de pression sont réglés pour une pression comprise entre10⁻³ et 5.10⁻² mmHg, pour obtenir un distillat comprenant principalement des lipides furaniques d'avocat.

Dans la demande internationale WO04/016106, les laboratoires Expanscience ont mis au point un procédé permettant d'obtenir avec un rendement élevé, un insaponifiable d'avocat riche en lipides furaniques, à savoir d'une teneur variant de 50 à 80%, présentant de faibles teneurs en produits lourds et en peroxydes. Ce procédé comprend les étapes successives suivantes :
(1) une étape de déshydratation contrôlée des avocats frais ou ayant subi des transformations préalables, réalisée à une température comprise entre -50°C et 75°C,
(2) une étape d'extraction de l'huile des fruits déshydratés,
(3) une étape, alternativement,
   - a. de traitement thermique de l'huile extraite à une température pouvant varier de 80 à 150°C, éventuellement sous atmosphère inerte, puis d'une étape de concentration de l'huile en sa fraction insaponifiable ou bien,
   - b. d'une étape de concentration de l'huile en sa fraction insaponifiable, puis d'un traitement thermique à une température pouvant varier de 80 à 150°C, éventuellement sous atmosphère inerte, suivie
(4) d'une étape de saponification et d'extraction de l'insaponifiable.

On entend plus généralement par déshydratation, effectuée à l'étape (1) du procédé, l'ensemble des techniques connues de l'homme de métier et qui permettent d'extraire l'eau d'un composé. Parmi ces techniques on préfèrele séchage en séchoirs ventilés, en couche mince et sous courant d'air chaud, à une température comprise entre 70 et 75°C, pendant 8 à 36 heures. L'étape de traitement thermique mise en oeuvre à l'étape (3) a. ou b. peut se faire en présence ou non d'un catalyseur acide, de préférence des alumines acides. L'étape de concentration de l'étape (3)a. ou (3)b. peut être une cristallisation à froid ou une distillation moléculaire.

De façon alternative, la préparation d'une fraction insaponifiable d'avocat composée d'alkyl furannes peut mettre en oeuvre des matières premières qui sont des co-produits issus des procédés d'extraction des huiles d'avocat. Notamment les huiles obtenues à partir des fruits frais, sans séchage préalable des fruits. Parmi ces co-produits on peut citer de manière non exhaustive : i) les phases grasses et ii) aqueuses issues des procédés dits de centrifugation, ou encore celles issus des procédés dits « enzymatiques », qui comprennent notamment une étape de pré-digestion enzymatique des cellules, végétales de la pulpe, afin de faciliter la libération des lipides du fruit. Les résidus solides de centrifugation (culots de centrifugeuse) qui sont extraits du débourbage des huiles brutes peuvent aussi constituer une matière première intéressante. Aussi, toujours de façon alternative, les pulpes congelées issues de fruits préalablement pelés et dénoyautés peuvent être utilisées. De même, les échappées de désodorisation des huiles d'avocat constituent aussi des sources d'insaponifiables d'avocat et d'alkyl furannes. En outre, les tourteaux d'avocat, co-produits lors de la pression mécanique à froid des fruits (frais ou séchés) ou de l'extraction liquide-solide de l'huile d'avocat à l'aide d'un solvant peuvent aussi constituer en l'état, une matière première alternative. Enfin, bien que pauvres en huile, les noyaux d'avocat peuvent potentiellement constituer une source de lipides d'avocat, et notamment d'alkyl furannes.

Dans le cadre de la présente invention, les alkyles furannes purifiés sont avantageusement utilisés selon une proportion comprise entre 0,001 et 100% en poids, et de préférence entre 0,5 et 60 % du poids total et de façon encore plus adaptée de 2% à 25% en poids par rapport au poids total du médicament. La quantité du médicament à administrer dépend de la gravité et de l'ancienneté de l'affection traitée. Naturellement, le médecin adaptera aussi la posologie en fonction du malade. Selon une variante avantageuse de l'invention, la dose thérapeutique par jour d'alkyles furannes selon l'invention est avantageusement comprise entre 10 mg et 30g/j et de préférence entre 60mg/j et 10 g/j et plus particulière de 100mg/j à 6g/j ou environ de 0,160 à 500mg/kg/j ou préférence, de 1mg/kg/j à 160mg/kg/j ou plus particulièrement de 16mg/kg/j à 100mg/kg/j.

En association avec le médicament, avantageusement avec un effet de synergie, on peut utiliser des traitements hypolipémiants, tels que des chélateurs de stérols (colestyramine), des fibrates (fénofibrate: agoniste PPAR-Alpha), des inhibiteurs de l'hmg-coA réductase (lovastatine, simvastatine), EPA/DHA (huile de poisson contenant des Omega 3, EPA = acide eicosapentaénoïque, DHA = acide docosahexaénoïque), la thérapie génique, des traitements de l'hypercliolestérolémie de diverses origines (primaires, monogéniques, polygéniques, alpha-lipoprotéinémie), des traitements des hyperlipidémiés mixtes, des traitements des hypertriglycéridémies, des traitements des dislipidémies. Les traitements généralement admis peuvent être la nutrition (régime méditerranéen est régulation des apports), la pharmacologie par les résines échangeuses d'ions, les fibrates, les statines, les huiles de poisson, les antioxydants, les phytostérols ou stanols.

En association avec le médicament avantageusement avec un effet de synergie, on peut utiliser des traitements antidiabétiques tels que des traitements oraux du diabète, l'insulinothérapie du diabète de type 2 et/ou des traitements divers tels que des capteurs de glucose et un pancréas artificiel. Comme exemple de traitement oral du diabète, on peut citer la stimulation de la sécrétion d'insuline (sulfamide hypoglycémiant ou apparenté (tolbutamide, carbutamide, glicazide, glimépiride, glipizide), dérivé de metformine, benfluorex), l'inhibition de l'alpha-glucosidase (acarbose et miglitol), le traitement de l'insulinorésistance (les thiazolidinediones (ou glitazone), tels que rosiglitazone et pioglitazone, ou les traitements de l'obésité comme les inhibiteurs de la recapture de la sérotonine (sibutramine), les inhibiteurs de la digestion des lipides (orlistat), les agonistes du récepteur Bêta3 adrénergique (augmente la lipolyse et la thermogénèse) ou l'augmentation de l'utilisation périphérique du glucose par réduction de l'oxydation des acides gras) ou encore l'insulino-sécrétion avec le GP1, la pramlintide, l'IGF1 et les dérivés de vanadium, les glinides.

En association avec le médicament avantageusement avec un effet de synergie, on peut utiliser des médicaments destinés au traitement des maladies cardiovasculaires (hypertension artérielle, coronaire, cérébrale et artériopathie secondaire) et/ou des complications bronchopulmonaires et rhumatologiques, cancer, atteinte du foie.

Le médicament peut en outre comprendre, en association, avantageusement avec un effet de synergie, des plantes ou aliments, hypoglycémiants, des oligo-élémentsnotamment le pidolate de chrome, des antioxydants, les agonistes PPARs naturels ou non, des insaponifiables stéroliques ou des produits pouvant en contenir (insaponifiables d'huiles végétales; notamment insaponifiables d'huile de soja, insaponifiables de beurres végétaux ou de matières butyreuses et leurs mélanges, insaponifiables de cires naturelles, insaponifiables d'extraits huileux, insaponifiables de co-produits huileux industriels, insaponifiables d'extraits de corps gras d'origine animale, insaponifiables d'huiles marines, insaponifiables d'extraits de la matière grasse lactique, insaponifiables de lipides extraits d'organismes unicellulaires, insaponifiables des lipides extraits des algues et organismes marins, etc), des stérols, des stanols, des phytostérols, des phytostanols, des tocophérols, des concentrats d'huiles de tournesol, de colza et/ou de palme, des acides gras en oméga 3, 6 ou 9, et des nutriments anti-graisse. Les insaponifiables « stéroliques » sont des insaponifiables dont la teneur en stérols, en méthylstérols et en alcools triterpèniques est comprise entre 20 et 95 % en poids, de préférence 45-65 % en poids, par rapport au poids total de l'insaponifiable. Les plantes hypoglycémiantes pouvant être utilisées dans le cadre de la présente invention en association avec les alkyles furannes sont avantageusement choisis dans le groupe constitué par Le fenugrec (*Trigonella graenum*)*,* l'acide corosolique (composé actif des feuilles de l'arbre *Lagestroemia speciosa*)*,* le *Gymnema syllvestre,* le jus de fruit de momordique (*Momormodica charantia*)*,* l'eucalyptus (*Eucalyptus globulus*), le *Panax ginseng,* les feuilles de myrtille (*Vaccinum myrtillus*).

Les alkyles furannes peuvent être utilisés en association, avantageusement avec un effet de synergie, avec des aliments ou des thérapies hyper glycémiantes pour rééquilibrer la glycémie tels que les antirétrovirus, les glucocorticoïdes, les immunosuppresseurs, IFN-Alpha, les stéroïdes sexuels, le THS, la pilule, les hormones de croissance, les sympathomimétiques, les médicaments cardio-vasculairés, les diurétiques, les Bêta-bloquants, les inhibiteurs calciques, les psychotropes.

Les alkyles furannes peuvent être utilisés en association, avantageusement avec un effet de synergie, avec des médicaments ou des aliments hyper lipémiants ou donnant une surcharge pondérale pour en diminuer le risque global et/ou en association avec des médicaments ou des aliments hypolipémiants pour diminuer le poids, influer : sur la glycémie et avoir un plus grand spectre d'efficacité.

Les plantes à mucilages peuvent jouer un rôle important dans le traitement des hyperglycémies, car elles ont un effet favorable sur la surcharge pondérale et les sécrétions excessives du pancréas. Les propriétés hydrophiles des fibres mucilagineuses, en formant un gel, diminuent l'assimilation des glucides et des lipides.

Les oligo-éléments pouvant être utilisés dans le cadre de la présente invention en association avec les alkyles furannes sont avantageusement choisis dans le groupe constitue par le magnésium, le chrome, le sélénium et leurs mélanges. Les antioxydants pouvant être utilisés dans le cadre de la présente invention en association avec les alkyles furannes sont avantageusement choisis dans le groupe constitué par le zinc, l'acide lipoïque, seul ou associé à la vitamine B12, les vitamines C, les flavonoïdes (thé vert,..), le beta-carotène, le lycopène ou la lutéine, les substances anti-glycation telles que la carnosine, la N-acétyl-cystéine, les isoflavones de soja, les protéines de soja.

On a démontré une augmentation du stress oxydant dans le diabète à travers une variété de mécanismes induits par l'augmentation des niveaux de sucre dans le sang. De plus, on pense que cette augmentation du stress oxydant contribue au développement des complications diabétiques comme des lésions sur les gros et petits vaisseaux (macre et micro-angiopathie).

Les médicaments à base de sulfonylurées et de glinides aident le pancréas à produire davantage d'insuline. Les sulfonylurées augmentent les niveaux d'insuline pendant plusieurs heures. Les glinides, qui se prennent avec les repars, augmentent les niveaux d'insuline pendant une durée plus courte que les sulfonylurées. Une glycémie basse est l'un des effets possibles de ces traitements. Les médicaments à base d'inhibiteurs des alpha-glucosidases ralentissent la digestion et l'absorption des amidons et des sucres, ce qui entraîne un ralentissement de la glycémie après les repas. Parmi les effets secondaires habituels entraînés par ces traitements, citons les gaz et le ballonnement ; cependant, les doses peuvent être augmentées de manière très graduelle pour réduire ces effets secondaires. Les médicaments à base de biguanides (metformine) travaillent principalement sur le foie. Ils empêchent essentiellement le foie de fabriquer de nouveaux sucres lorsque cela n'est pas nécessaire. Les médicaments au biguanide peuvent occasionner des effets, secondaires, dont les plus courants sont les maux d'estomac et la nausée. Pour réduire les effets secondaires, il est conseillé de prendre les biguanides avec les repas.

Les médicaments à base d'activateurs de la sensibilité à l'insuline ou thiazolidinediones (TZD, pioglitazone, rosiglitazone), qui sont des agonistes PPARs, sont des nouveaux types de traitement. Ce nouveau type de traitement oral est décrit comme un activateur de la sensibilité à l'insuline, ou encore TZD. Ces médicaments traitent la résistance à l'insuline, l'une des principales causes de diabète. La résistance à l'insuline est une situation dans laquelle l'organisme n'utilise pas correctement l'insuline qu'il produit. En réduisant la résistance à l'insuline, un TZD permet à l'insuline, fabriquée ou administrée, de fonctionner plus efficacement, et contribue ainsi à réduire l'augmentation dangereuse de la glycémie. Parmi les effets secondaires possibles des TZD, notions la prise de poids, la formation d'oedèmes (rétention d'eau) et une faible anémie.

Les médicaments hypolipémiants pouvant être utilisés en association avec les alkyles furannes sont avantageusement choisis dans le groupe constitué par les médicaments hypolipémiants de la famille des statines ou de la famille des fibrates (agonistes de PPARα). Les médicaments anti-obésité pouvant être utilisés en association avec les alkyles furannes sont avantageusement choisis dans le groupe constitué par l'orlistat (Xenical ® et la sibutramine (Reductyl® ou Sibutral®). Les anciens médicaments coupe-faim (les amphétamines, la fenfluramine) ne sont plus utilisés car ils avaient trop d'effets secondaires. En France, seul deux médicaments ont reçu l'autorisation de mise sur le marché pour le traitement de l'obésité: l'orlistat (Xenical®) et la sibutramine (Reductyl® ou Sibutral®). L'orlistat (Xenical®) est un médicament qui a pour effet d'entraver l'enzyme responsable de l'absorption des graisses dans le tube digestif. Il empêche ainsi l'absorption d'environ 30% des graisses alimentaires (triglycérides), ce qui équivaut à une réduction calorique de 150 à 200 calories/jour pour un apport journalier de 1800 calories. Les graisses non absorbées sont éliminées dans les selles. La sibutramine (Reductyl® ou Sibutral®) est une molécule qui agit sur des neuromédiateurs du cerveau qui jouent un rôle au niveau du contrôle de la prise alimentaire. Elle a pour propriété de renforcer la sensation de satiété et diminue la prise alimentaire.

Les nutriments anti-graisse pouvant être utilisés dans le cadre de la présente invention en association, avantageusement avec un effet de synergie, avec les alkyles furannes sont avantageusement choisis dans le groupe constitué par des nutriments bloqueur de l'absorption des graisses, tels que le chitosan, qui est une fibre extraite de l'exosquelette des crustacés, des nutriments capables d'augmenter la thermogénèse brûleur de graisse ») tels que l'éphédrine (herbe chinoise Ma Huang); la caféine, la théine et le citrus aurantium, le CLA (Acide linoléique conjugué issu de carthame préférentiellement), les huiles de poisson s riche en oméga 3, de palme de cactus capteurs de lipides, les extraits sec des nutriments capables de réguler l'appétit (« coupe faim ») tels que la L-phénylalanine et la L-tyrosine, des nutriments capables de réguler la glycémie, tels que des minéraux, par exemple le chrome ou le vanadium ou le magnésium, ou l'herbe ayurvédique Gymnema sylvestre, des inhibiteurs de la lipogénèse, tels que l'acide hydroxycitrique extrait du Garcinia cambodgia et des nutriments capables de transporter des graisses tels que la L-carnitine.

La présente invention décrit également l'utilisation d'une composition cosmétique et/ou d'une composition nutraceutique comprenant un ou plusieurs alkyles furannes, synthétiques ou naturels, pour le traitement cosmétique des troubles liés à une surcharge pondérale.

La présente invention a pour objet l'utilisation d'un ou plusieurs alkyles furannes, synthétiques ou naturels, pour le traitement cosmétique de la cellulite.

La surcharge pondérale se caractérise, dans le cadre de la présente invention, par un surpoids, par rapport au « poids idéal », non pathologique. Le traitement cosmétique permet de perdre ou d'affiner des rondeurs localisées superflues mais ne s'identifie pas à un traitement thérapeutique.

L'application topique d'une composition cosmétique comprenant un ou plusieurs alkyles furannes, synthétiques ou naturels, peut s'avérer particulièrement avantageuse chez la femme enceinte ou la femme ayant accouché depuis moins de 6 mois. La présente invention décrit également une méthode de traitement cosmétique pour favoriser l'amincissement et notamment pour lutter contre la surcharge pondérale localisée chez la femme enceinte ou la femme ayant accouché depuis moins de 6 mois. En effet, l'un des avantages des compositions utilisables dans le cadre de la présente invention est de ne pas nécessiter la présence d'alcool pour la formulation, qui est contre indiqué pour les femmes enceintes et en allaitement du fait de sa toxicité. En effet, la caféine, actif amincissant très couramment utilisé nécessite une solubilisation dans l'alcool, qui est ainsi évitée dans le cadre de la présente invention.

Par l'expression « amincissement » ou « lutte contre la surcharge pondérale localisée», on entend une action permettant d'éviter ou tout au moins de réduire la formation de graisses sous-cutanées telles que décrites précédemment. Cette action se traduit notamment par une diminution des surcharges ou réserves disgracieuses, par un affinement de la silhouette, par une accélération de l'élimination des excédents, par une meilleure définition du contour de corps ou encore une silhouette resculptée. Par méthode de «traitement cosmétique pour lutter contre la surcharge pondérale localisée » on entend, la mise en oeuvre d'un traitement cosmétique permettant de mesurer de manière visible l'action décrite ci-dessus. Par exemple, une composition cosmétique topique comprenant un ou plusieurs alkyles furannes, synthétiques ou naturels, utilisée selon l'invention peut être appliquée sur les zones de la peau susceptibles de former ces surcharges pondérales localisées, à savoir des zones où ces surcharges sont déjà formées ou en cours de formation

Dans le cadre de l'invention, lesdits alkyles furannes, utilisés comme principes actifs dans la composition cosmétique et/ou nutraceutique pour le traitement cosmetique de la cellulite, sont tels que définis précédemment.

Dans le cadre de la présente invention, les alkyles furannes sont avantageusement utilisés selon une proportion, comprise entre 0,001 et 25% en poids, par rapport au poids total de la composition cosmétique, encore plus avantageusement 1 et 10% en poids.

Le complément alimentaire peut comprendre en poids 0,001 à 100% d'alkyles furannes selon l'invention. Dans un aliment, les concentrations utilisables en alkyl furannes purs peuvent être de 0,001 à 10 % en poids, par rapport au poids total de l'aliment.

La composition cosmétique et/ou la composition nutraceutique selon l'invention peut en outre comprendre, en association, avantageusement avec un effet de synergie, des plantes hypoglycémiantes, des oligo-éléments, des antioxydants, des nutriments anti-graisse, des insaponifiables stéroliques ou des produits pouvant en contenir, des stérols, des stanols, des phytostérols, des phytostanols, des tocophérols, des concentrats d'huiles de tournesol, de colza et/ou de palme, des acides gras en oméga 3, 6 ou 9, tels que décrits précédemment.

Le médicament et la composition cosmétique peuvent être formulés sous la forme de différentes préparations adaptées à une administration orale ou topique. La composition nutraceutique selon l'invention peut être formulée sous la forme de différentes préparations adaptées à une administration orale ou sous la forme d'un aliment.

Lorsque le médicament, la composition cosmétique ou nutraceutique sont administrés per os, ils peuvent être administrés sous formes unitaires ou multidoses d'administration en mélange avec des supports pharmaceutiques, cosmétiques ou alimentaires adéquats connus de l'homme du métier. Les formes unitaires d'administration appropriées comprennent notamment les comprimés éventuellement sécables, les gélules, les poudres, les granulés et les solutions ou suspensions orales. Les formes multidoses d'administration appropriées comprennent notamment les gouttes buvables, les émulsions et les sirops. Lors de la préparation de comprimés, les alkyles furannes selon l'invention sont mélangés avec un véhicule acceptable tel que notamment la gélatine, le talc, l'amidon, le lactose, le stéarate de magnésium, la gomme arabique ou leurs analogues. Les comprimés peuvent éventuellement être enrobés, c'est-à-dire recouverts de plusieurs couches de substances diverses telles que du saccharose, afin de faciliter la prise ou la conservation. Les comprimés peuvent encore présenter une formulation plus ou moins complexe destinée à modifier la vitesse de libération du principe actif. La libération du principe actif dudit comprimé peut être accélérée, ralenti ou retardée en fonction de l'absorption désirée. On obtient une préparation en gélules en mélangeant les alkyles furannes selon l'invention avec un diluant. Le mélange ainsi obtenu est versé dans des gélules molles ou dures. Une préparation sous forme de sirop peut contenir les alkyles furannes selon l'invention conjointement avec un édulcorant, avantageusement acalorique, un agent donnant du goût et un colorant approprié. Les poudres ou les granulés dispersibles dans l'eau peuvent comprendre les alkyles furannes selon l'invention en mélange avec des argents de dispersion ou des agents mouillants, des agents de mise en suspension, comme la polyvinypyrrolidone, ou des édulcorants ou des correcteurs de goût.

Selon une variante de la présente invention, le médicament et la composition cosmétique peuvent être destinés à un usage externe topique. Ils peuvent par ailleurs contenir un support pharmaceutiquement ou cosmétiquement acceptable. Le médicament ou la composition cosmétique selon l'invention peuvent se présenter sous toutes les formes galéniques usuellement utilisées pour une application externe topique. Avantageusement selon la présente invention, le médicament ou la composition cosmétique se présentent sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, ou d'une dispersion d'huile dans une phase aqueuse à l'aide de sphérules, les sphérules pouvant être des nanoparticules polymériques telles que les nanosphères et les nanocapsules ou mieux des vésicules lipidiques de type ionique et/ou non-ionique. Le médicament ou la composition cosmétique peuvent être plus ou moins fluides, se présenter sous forme de crème blanche ou colorée, de pommade, de lait, de lotion, d'onguent, de sérum, de pâte, de mousse, d'aérosol ou de stick.

Le médicament ou la composition cosmétique selon la présente invention peuvent par ailleurs contenir les adjuvants habituels dans le domaine pharmaceutique et/ou cosmétique, tels que les gélifiants hydrophiles ou lipophiles, les actifs hydrophiles ou lipophiles, les conservateurs, les antioxydants, les solvants, les parfums, les charges, les filtres chimiques ou minéraux, les pigments, les agents chélateurs, les absorbeurs d'odeur, les eaux thermales et les matières colorantes. Les quantités de ces différents adjuvants sont celles classiquement utilisées en pharmaceutique et/ou en cosmétique, et par exemple de 0,01 % à 20% en poids, par rapport au poids total du médicament ou de la composition cosmétique. Ces adjuvants, selon leur nature, peuvent être introduits dans la phase grasse, dans la phase aqueuse, dans les vésicules lipidiques et/ou dans les nanoparticules.

Lorsque le médicament ou la composition cosmétique selon la présente invention est une émulsion, la proportion de la phase grasse peut aller de 5% à 80% en poids, et de préférence de 5% à 50% en poids, par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30% en poids, et de préférence de 0,5 % à 20 % en poids, par rapport au poids total de la composition. Parmi les huiles utilisables selon la présente invention, on peut citer notamment les huiles minérales, d'autres huiles d'origine végétale (huile d'abricot, huile de tournesol, de prune), les huiles d'origine animale, les huiles de synthèse, les huiles siliconées et les huiles fluorées (perfluoropolyéthers). Des alcools gras, tels que l'alcool cétylique, des acides gras, ou des cires telles que la cire d'abeilles peuvent également être utilisées en tant que matières grasses selon la présente invention. Parmi les émulsionnants et coémulsionnants utilisables selon la présente invention, on peut citer notamment les esters d'acide gras et de polyéthylène glycol, tels que le stéarate de PEG-40 ou le stéarate de PEG-100, les esters d'acide gras et de polyol, tels que le stéarate de glycéryle et le tristéarate de sorbitane. Parmi les gélifiants hydrophiles utilisables selon la présente invention, peuvent notamment être cités les polymères carboxyvinyliques (carbomère), les copolymères acryliques tels que les copolymères d'acrylate/alkylacrylate, les polyacrylamides, les polysaccharides, les gommes naturelles et les argiles. Parmi les gélifiants lipophiles, peuvent notamment être cités les argiles modifiées telles que les bentones, les sels métalliques d'acides gras, la silice hydrophobe et les polyéthylènes.

Les modes d'administration, les posologies et les formes galéniques optimales des composés et compositions selon l'invention peuvent être déterminés selon les critères généralement pris en compte dans l'établissement d'un traitement pharmaceutique, vétérinaire, cosmétique ou nutraceutique, adapté à un patient ou à un animal comme par exemple l'âge ou le poids corporel du patient ou de l'animal, la gravité de son état général, la tolérance au traitement, les effets secondaires constatés, le type de peau.

Selon une variante de la présente invention, on peut incorporer, sans aucune restriction, les alkyle furannes de la présente invention dans la nourriture, les boisons et les nutraceutiques, y compris dans les produits laitiers (fromages, beurre, lait et autres breuvages lactés, mélanges et pâtes à tartiner à base de produits lactés, crèmes glacées et yaourt), les produits à base de graisse (margarines, pâtes à tartiner, mayonnaises, matières grasses pour cuisson, huiles à frire et vinaigrettes), les produits à base de céréales composés de graines tels que le pain et les pâtes, que ces aliments soient cuisinés, cuits au four ou transformés, les_confiseries (chocolat, bonbons, chewing gums, desserts, nappages, sorbets, glaçages et autres garnitures), les boissons alcoolisés ou non (sodas et autres boissons non alcoolisées, jus de fruits, compléments diététiques, substituts de repas sous forme de breuvage comme ceux vendus sous la marque Boost™ and Ensure™) et les produits divers (oeufs, aliments transformés comme les soupes, les sauces toute prêtse pour pâtes, plats préparés et autres produits du même genre). La composition de la présente invention peut être incorporée directement et sans autre modification dans la nourriture, les nutraceutiques ou les breuvages et ce grâce à des techniques comme le mixage, l'infusion, l'injection, le mélange, l'absorption, le pétrissage et la pulvérisation. Il est aussi possible pour le consommateur d'appliquer directement la composition sur la nourriture ou dans le breuvage avant ingestion. Ces modes d'administration sont simples et économiques.

Les exemples suivants illustrent la présente invention.

### Exemple comparatif 1: Cosmétique Anti-tache visage diabétique (Quantité : 100g)

| | % |
|---|---|
| Eau | 64,83 |
| Peptide de lupin | 7 |
| Insaponifiable d'avocat furanique | 2 |
| Butylene glycol | 3 |
| Alcool cétylique | 6 |
| Alkyl lactate C12-13 | 2 |
| Huile minérale | 7 |
| Ceteth 20 | 2 |
| Acide stéarique | 1 |
| Extrait de mûrier blanc | 1 |
| Acétate de Tocophérol | 0,50 |
| Proplène glycol | 0,56 |
| EDTA dissodique | 0,52 |
| Triéthanolamine | 0,80 |
| Conservateur | 0,30 |
| Acide citrique | 0,25 |
| Methyl paraben | 0,11 |
| Steareth -20 | 1 |
| Metabisulfite de sodium | 0,05 |
| Sulfite de sodium | 0,05 |
| Propyl paraben | 0,03 |

### Exemple comparatif 2 : Crème pour le confort de la peau des diabétiques (Quantité : 100g)

| | % |
|---|---|
| Vaseline officinale | 6,00 |
| Huile palmiste hydrogénée | 4,00 |
| Caprylo caprate de glycérol | 2,00 |
| Sucro ester 7 (Distérate sucrose)) | 6,00 |
| Squalane | 1,00 |
| Cire de candelilla | 2,00 |
| Sucro ester 11 (Stéarate sucrose) | 0,50 |
| 2-Alkyl furane | 2,00 |
| Concentrat de tournesol | 2,00 |
| Glycérol | 5,00 |
| Glucodextrine | 1,00 |
| Trométamine | 0,01 |
| Gomme xanthane | 0,20 |
| Hydroxyméthylglycinate A | 0,60 |
| Acide citrique | 0,32 |
| Cyclométhiconol | 5,00 |
| Céramide/Cholesiérol | 0,60 |
| Eau purifiés | 5,00 |

### Exemple comparatif 3: Crème protectrice de la peau diabétique (Quantité : 100g)

| | % |
|---|---|
| Squalane | 1,00 |
| Erythrityl ester | 4,00 |
| Decyl pentanoate | 4,00 |
| Cetearyl glucoside | 2,00 |
| Lauryl ether 23 OE | 1,00 |
| Cutina CBSV | 1,00 |
| Cire d'abeille | 0,50 |
| Myrista myristyle | 1,00 |
| Conservateur | 0,30 |
| Vaseline épaisse | 5,00 |
| Squalane gelifiée | 3,00 |
| **Huile d'avocat furanique** | **10,00** |
| Eau purifiée | 56,51 |
| Phenoxyéthanol | 0,80 |
| EDTA de sodium | 0,10 |
| Acide citrique | 0,14 |
| Sorbate de potassium | 0,45 |
| Glycérine | 5,00 |
| Epaississant | 0,50 |
| Lessive de soude | 0,30 |
| Polyacrylamide gel 60° | 1,00 |
| Acétate vitamine E 35° | 0,50 |
| Parfum | 0,50 |
| Peptide de lupin | 1,00 |
| Cyclométhyconol | 7,00 |
| Silice TIO2 | 1,00 |
| Génisteine 85% | 0,10 |
| PEG 300 | 0,90 |

### Exemple comparatif 4 : Crème pour les pieds (Quantité : 100g)

| | % |
|---|---|
| Montanov | 68 3 |
| Amphisol K | 0,50 |
| Miglyol 812 | 6 |
| Conservateur | 0,30 |
| Beurre de karité | 1 |
| **Huile d'avocat furanique** | 1 |
| **2-Alkyl furane** | 5 |
| EDTA Na₂ | 0,10 |
| Acide citrique | 0,01 |
| Conservateur | 0,40 |
| Butylène glycol | 1 |
| Gélifiant | 0,25 |
| Lessive de soude | 0,4 |
| Gluconate manganèse | 0,05 |
| Zinc de sel | 0,10 |
| Eau purifiée | QSP |

### Exemple comparatif 5 : Formules pour la régulation de la glycémie et de l'hypercholestérolémie

❖ hypercholestérolémies et hypertriglycéridémies

| | |
|---|---|
| Fénofibrate | 200 mg |
| **Insaponifiable d'avocat furanique** | **100 mg** |
| Excipient QSP pour une gélule banderolée | QS |

❖ Capsule molle nutraceutique régulatrice de la prise de poids et de la glycémie

| | |
|---|---|
| Acides gras polyinsaturés de poisson | 1 000 mg |
| **Huile d'avocat furanique** | **500** |
| Chrome | 1 |
| Ananas | 50 |
| Pectine de pomme | 50 |
| Excipient | QS |

❖ Médicament hypolipémiant et anti-diabétique

| | |
|---|---|
| Atorvastatine | 10 à 40 mg |
| **2-Alkyl furane** | **33 à 100 mg** |
| Excipient | QS |

❖ Traitement de l'obésité

| | |
|---|---|
| Mucilage (alginate de sodium) | 500 mg |
| Huile de poisson | 500 mg |
| **Insaponifiable d'avocat furanique** | **100 mg** |
| Sel de zinc | 1 mg |
| Orthosiphon | 325 mg |
| Fucus | 50 mg |
| Excipient capsule molle | QS |

### Exemple 6 : Poudre nutraceutique (quantité : 100g)

| | |
|---|---|
| **Insaponifiable d'avocat furanique absorbé sur cyclodextrine** | **50,00** |
| Amidon 1500 | 49,40 |
| Stéréate de magnésium | 0,60 |

### Exemple 7 : Gel crème amincissant (Quantité : 100g)

| | % |
|---|---|
| Carbopol Etd 2020 | 0,6 |
| Gomme xanthane | 0,15 |
| Génisteine 85% | 0,1 |
| Alcool + Caféine | 3,6 |
| NaOH | 0,001 |
| Conservateur | 0,9 |
| **Insaponifiable d'avocat** | **2** |
| Glucodextrine | 2 |
| Parfum | 0,7 |
| Silicone | 0,3 |
| Eau purifiée | QSP |

### Exemple comparatif 8: Lingettes hygiène des pieds diabétique (Quantité : 100g)

| | % |
|---|---|
| Poloxamer 184 | 1,0000 |
| Parfum | 0,2000 |
| Eau purifiée | 91,1550 |
| PEG - 32 | 4,0000 |
| Conservateur | 1,0000 |
| Chlorhexidine | 0,1500 |
| Phenoxyéthanol | 0,1000 |
| Allantoine | 0,2000 |
| **2-Alkyl furane** | 1,0000 |
| Solubilisant | 1,0000 |
| Trométhamine | 0,1950 |

### Exemple 9 : Etude des effets des 2-alkylesfurannes sur le métabolisme :

### 1) Produit testé :

On teste la fraction furanique purifiée de l'insaponifiable d'avocat, commercialisée par les Laboratoires Expanscience sous la dénomination Avocadofurane® (AV102) qui contient 98% en poids de 8-11-cis-cis-heptadécadienyl-2-furanne.

### 2) Protocole expérimental

Nous avons utilisé des souris adultes (âgées de 6 semaines) de sexe masculin engraissées avec un régime riche en matières grasses et en sucres (HF/HS high fat/high sucrose). L'alimentation avec le régime HF/HS commence sur des souris âgées de 6 semaines et continue tout au long de la durée de l'étude. L'étude est menée suivant 2 axes :
- prévention : l'administration du produit commence dès le début de l'alimentation avec le régime riche en sucres et en matière grasse ;
- intervention (curatif) : le produit est administré 12 semaines après le début du régime riche en sucres et en matière grasse.
Durée totale du traitement (prévention et intervention) : 23 semaines.

Chaque groupe expérimental comprend 10 souris. Dans le cadre de l'étude, on met en place 8 groupes expérimentaux :
- 2 groupes de contrôle : contrôle négatif : les souris reçoivent une alimentation normale
   contrôle positif: les souris reçoivent une alimentation HF/HS
- 2 groupes de référence : régime HF/HS + TZD (thiazolidine dione (Rosiglitazone), agoniste PPARα) à 10mg/kg/jour
   régime HF/HS puis au bout de 12 semaines TZD à 10mg/kg/jour
- 2 groupes de traitement : régime HF/HS + 0,1% en poids AV102, par rapport au poids total de la diète
   régime HF/HS + AV102 à une concentration massique de 0,25%

Chaque étude démarre à J-7 (soit 7 jours avant le début du régime alimentaire riche en sucre et en matière grasse) avec une analyse de la tolérance au glucose au moyen soit d'un test de tolérance au glucose intra-péritonéal pour une partie des animaux (n=5) soit d'un test de sensibilité à l'insuline intraveineux pour l'autre partie des animaux (n=5). A J=0, des prélèvements sanguins sont effectués afin d'obtenir les valeurs en conditions basales de tous les paramètres lipidiques et lipoprotéiniques puis les souris sont réparties de manière aléatoire, indépendamment de leur poids. Le poids de chaque souris et la quantité de nourriture absorbée sont mesurés deux fois par semaine. Aux semaines 3, 8, 15 et 23, le sang est collecté suite à une diète, qui dure toute la nuit. Aux semaines 12 et 18, une partie des animaux (n=5) est soumise à un test de tolérance au glucose intra-péritonéal, l'autre partie (n=5) est soumise à un test de sensibilité à l'insuline intraveineux. Semaine 19, les matières fécales sont collectées pour analyse de la composition en lipides (mesure des triglycérides). Semaine 14 (aspect préventif) et semaine 20 (aspect préventif et curatif), une analyse Dexascan est réalisée sur les animaux anesthésiés pour mesure leur densité minérale osseuse, le poids du squelette et la teneur en graisse. Semaine 21 et 22, la dépense énergétique est mesurée par calorimétrie indirecte. A la fin de l'étude (semaine 23), les animaux sont sacrifiés et on récupère leurs foies, intestins, tissus adipeux et muscles, qui sont pesés, congelés et conservés à une température de -80°C pour des analyses supplémentaires. Le foie et les tissus adipeux sont également préparés en vue d'analyses histologiques.

Aux semaines 3, 8, 15 et 23, on réalise les analyses sanguines suivantes : lipides contenus dans le plasma (cholestérol total, triglycérides, cholestérol HDL (« bon » cholestérol, High Density Lipoprotein), cholestérol LDL (« mauvais » cholestérol, Low Density Lipoprotein), acides gras libres) ; glucose et insuline ; leptine; TNF-α; Transaminases et phosphatase alcaline (toxicité hépatique). Puis, on analyse : le profil des lipoprotéines (par chromatographie par exclusion de taille) ; Apolipoprotéine A1 ; la composition lipidique du foie (triglycérides du foie, cholestérol et acides gras libres) ; analyse histologique du foie et des tissus adipeux ; coloration des lipides accumulés dans le foie.

### 3) résultats :

Les doses d'AV102 théoriquement absorbées par les souris sont de 160 mg/kg/j d'AV102 à 0,1% (souris 25g, mangeant 4g de nourriture/j) et de 400 mg/kg/j (souris 25g, mangeant 4g de nourriture/j). Les doses effectivement ingérées par les souris en phase d'intervention et en phase prévention sont données dans le tableau 2 suivant :

**Tableau 2 : doses d'AV102 ingérées par les souris en intervention ou en prévention**

| - | Phase intervention | Phase prévention |
|---|---|---|
| AV102 (0,1%) | 94 +/-10 mg/kg/j | 136 +/- 24 mg/kg/j |
| AV 102 (0,25%) | 183 +/- 42 mg/kg/j | 314 +/- 72 mg/kg/j |

### 1. Prise de nourriture et poids

On a vérifié que les différents traitements n'influaient pas sur l'alimentation normale des souris. On a également constaté une diminution du poids des souris soumises au régime HF/HS avec AV102 (0,1% ou 0,25%). Les résultats sont donnés dans le tableau 3 suivant :

**Tableau 3 : prise de nourriture- poids des souris soumises au régime HF/HS**

| | Prévention | | Intervention | |
|---|---|---|---|---|
| | Nourriture | Poids souris | Nourriture | Poids souris |
| TZD (10 mg/kg/j) | ↓ | - | ↓ | - |
| AV 102 (0,1%) | - | ↓ (14 semaines) | - | ↓ (4 semaines) |
| AV102 (0,25%) | ↓ | ↓↓↓(1 semaine) | ↓ | ↓↓↓ (3 jours) |

Dans le tableau 4 suivant, on donne le ratio du poids des souris soumises aux différents régimes HF/HS (diète normale/régime HF/HS seul/ régime HF/HS avec TZD et régime HF/HS avec AV 102 à 0,1% ou 0,25%) après 20 semaines de traitement par rapport au poids de ces souris avant le traitement (semaine 0 en prévention, semaine 12 en intervention)

**Tableau 4 : poids des souris soumises au régime HF/HS**

| | Contrôle négatif | Contrôle positif | TZD 10mg/kg/j | AV102 0,1% | AV102 0,25% |
|---|---|---|---|---|---|
| Prévention : Ratio semaine 20/semaine 0 | 1,33 | 1,54 | 1,66 | 1,37 | 1,05 |
| Intervention : Ratio semaine 20/semaine 12 | 1,03 | 1,12 | 1,17 | 1,02 | 0,76 |

### Prise de nourriture

Groupe prévention : on observe une consommation quasi normale des souris soumises au régime HF/HS avec AV102 0,1% (± 3,7 grammes). On observe une consommation normale jusqu'à J56 puis une légère diminution et un retour à la normale en fin de traitement pour les souris soumises au régime HF/HS avec AV102 0,25%, sans impact sur les résultats (consommation moyenne 3 g ; idem TZD).

Groupe intervention : on observe un impact des principes actifs et surtout de l'AV102 0,25% (4 g groupe contrôle, 3 g TZD AV102 0,1 %, 2,5g AV102 0,25%).

### Prise de poids

Groupe prévention : chez les souris soumises au régime HF/HS avec AV102 à 0,1%, on observe une diminution de 11% de poids desdites souris ; on a ainsi un retour à un poids normal. Chez les souris soumises au régime HF/HS avec AV102 à 0,25%, on observe une diminution de 32 % du poids desdites souris: en fin de traitement, le poids de ces souris est inférieur au poids des souris sous diète normale.
→ AV102 s'oppose à la prise de poids et fait fortement maigrir sous diète riche en lipides et sucres, et ce dès les premiers jours de traitement pour une concentration, de 0,25%.

Groupe intervention : Soumises au régime HF/HS seul, les souris grossissent de 12%. L'ajout de TZD à l'alimentation ne fait pas maigrir les souris en surpoids. L'ajout d'AV102 à 0, 1 % dans l'alimentation fait maigrir les souris de 11 % avec un retour à un poids normal. L'ajout d'AV102 à 0,25% dans l'alimentation fait maigrir les souris de 33% soit une perte conséquente de poids par rapport à la normalité.
→ AV102 fait maigrir les souris initialement en surpoids suivant un effet dose/temps (quasi immédiat) qui peut conduire à un fort amaigrissement

### 2. Paramètres sanguins

### ■ Lipides

On a analysé les différentes les taux sanguins de cholestérol et de ses différentes fractions en utilisant une méthode HPLC.

**Tableau 5:**

| | **TZD (10 mg/kg/j)** | | **AV102 0,1%** | | **AV102 0,25%)** | |
|---|---|---|---|---|---|---|
| PREVENTION | cholestérol total | ↓↓ (3S***) | cholestérol total | ↑ | cholestérol total | ↑ |
| | LDL | - | LDL | ↑↑ (3S***) | LDL | ↑↑ (3S***) |
| | HDL | ↓↓ (3S***) | HDL | - | HDL | - |
| | TG | ↓ | TG | ↓ | TG | ↓↓↓↓ (3S***) |
| | FAA | - | FAA | - | FAA | - |
| | **TZD (10 mg/kg/j)** | | **AV102 0,1%** | | **AV102 0,25%)** | |
| INTERVENTION | cholestérol total | ↓↓ (3S***) | cholestérol total | ↓ | cholestérol total | ↑ |
| | LDL | ↓ | LDL | ↑↑ (3S**) | LDL | ↑↑ (11S**) |
| | HDL | ↓↓ (3S***) | HDL | ↑↑ (3S***) | HDL | - |
| | TG | - | TG | - | TG | ↓↓↓↓ (11S***) |
| | FAA | - | FAA | ↓↓ (11S***) | FAA | ↓↓ (11S***) |

| | | | | | | |
|---|---|---|---|---|---|---|
| S = semaine - TG= triglycerides - FAA= acide gras libres | | | | | | |

Groupe prévention : une diminution significative du cholestérol total, des taux de cholestérol HDL est observée après 3, 8, 15 et 23 semaines de traitement avec TZD. Les taux de cholestérol LDL (low density lipoprotéine = lipoprotéine de faible densité) ont également tendance à diminuer dans ce groupe. Cette augmentation est également constatée dans des profils lipoprotéiniques plasmatiques qui montrent une diminution des fractions du cholestérol HDL, LDL et VLDL (very low density lipoprotéine = lipoprotéine de très faible densité) aux semaines 8 et 15.

Le cholestérol total augmente chez les souris traitées avec AV102 à 0, 1 % après 3 et 15 semaines de traitement et chez les souris traitées avec AV102 à 0,25% après 3, 15 et 23 semaines de traitement. Les profils lipoprotéiniques, semaines 8 et 15, montrent une augmentation de la fraction cholestérol HDL et une diminution des fractions cholestérol LDM et VLDL chez les souris traitées avec AV102 aux deux doses.

Les taux plasmatiques de triglycérides sont significativement diminués après 8 semaines de traitement avec TZD et AV102 à 0, 1 %, mais aucune diminution supplémentaire n'est observée après 15 et 23 semaines de traitement avec ces produits. Une diminution significative des taux de cholestérol est observée après 3, 8, 15 et 23 semaines de traitement avec AV102 à 0,25%.

Les taux d'acides gras libres ne varient pas de manière significative.

Groupe intervention : une diminution du cholestérol total, des taux de cholestérol HDL et LDL est observée après 3 et 11 semaines de traitement avec TZD. Les profils lipoprotéiniques de ces souris montrent également une diminution des fractions de cholestérol HDL et VLDL après 3 semaines de traitement.

Le cholestérol total a tendance a augmenté chez les souris traitées pendant 3 et 11 semaines avec AV102 au deux doses. les profils lipoprotéiniques des souris traitées avec AV102 à 0,25% durant 11 semaines montrent une augmentation de la fraction cholestérol LDL et une diminution des fractions cholestérol HDL et VLDL.

Les taux de triglycérides sont significativement diminués chez les souris traitées avec AV102 à 0,25% après 3 et 11 semaines. Aucun changement du taux de triglycérides n'est observé avec TZD et AV102 à 0,1%.

Les taux d'acides gras libres diminuent après 10 semaines de traitement avec AV102 aux deux doses.

Ainsi, l'Avocadofurane, surtout à 0,25% :
- prévient l'élévation et diminue très significativement les triglycérides en phase de prévention ou d'intervention
- augmente le cholestérol total, le HDL, diminue (LDL, VLDL)

Le TZD n'a pas ou peu d'action sur les TG, mais il diminue le cholestérol total

### ■ Glucose

### A- Analyse sanguine glucose

Groupe prévention : Les taux de glucose sont diminués dans chaque groupe traité après 3 semaines. Semaines 8, 15 et 23, cette diminution est toujours observée pour les souris traitées avec AV102, aux deux doses de traitement, par contre cette diminution n'est plus observée pour les souris traitées avec TZD.

TZD diminue l'hyperglycémie «constitutive» avec un retour à la normale. AV102, à la dose de 0,25%, intervient de façon très significative et de façon plus efficace que le TZD.

Si on regarde le pourcentage d'évolution de la glycémie de différents groupes par rapport à la glycémie du groupe diète normale à 3 et 23 semaines, l'ensemble des principes actifs a une action sur l'hyperglycémie provoquée par la diète riche. AV102 est le composé dont l'activité effet dose se prolonge tout au long de l'étude de façon très significative.

Groupe intervention : les taux de glucose sont significativement diminués après 3 et 11 semaines de traitement avec TZD et AV102 à 0,25 %.

### B- Résistance au glucose (IPGTT)

IPGTT = test de diagnostic du diabète avec la glycémie à jeun (Intraperitoneal Glucose Tolerance Test).

On a remarqué une prévalence de la perturbation de la tolérance au glucose très élevée chez les enfants obèses. De plus, cette prévalence est souvent accompagnée par une résistance à l'insuline.

Protocole :
- souris à jeun
- mesure de la glycémie à T0
- injection intra-péritonéale de glucose
- mesure de la glycémie 15, 30, 45, 60, 90, 120 et 180 min après l'injection
- cinétique réalisée semaines 1, 12 et 18 en prévention et 18 en intervention

Groupe prévention : les résultats du test IPGTT montrent une diminution du AUC (area under curve = aire sous la courbe) chez les souris soumises au régime HF/HS + AV102 à une concentration de 0,25 %, aux semaines 12 et 18. Il n'y a pas de diminution significative du AUC chez les souris soumises au régime HF/HS avec TZD.

Le régime HF/HS augmente la résistance au glucose. TZD : diminue la résistance au glucose : courbe intermédiaire entre HF/HS et diète normale à 12 semaines. A 18 semaines, TZD annule complètement les effets de HF/HS avec un retour à une cinétique normale. AV102 à 0, 1 % et 0,25% : diminution significative et de façon plus importante que le TZD de la résistance au glucose vs HF/HS à 12 et 18 semaines. A cette date, la courbe est en dessous de la cinétique normale.

Groupe intervention : les résultats du test IPGTT ont le même profil que les résultats observés dans le groupe prévention : une diminution du AUC est observée chez les souris traitées avec AV102 à 0,25% durant 6 semaines.

Le régime HF/HS induit une forte résistance au glucose. TZD n'intervient pas sur la résistance au glucose établie et conférée par un régime riche. AV102 à 0,1% et 0,25% interviennent de façon très significative et effet dose sur cette résistance.

### C- Sensibilité à l'insuline (groupe prévention)

Les diabétiques ont une augmentation de la résistance à l'insuline qui entraîne une hyperglycémie. IPIST = Intra Insuline Sensitive Test = test de la sensibilité intra péritonéal à l'insuline.

Protocole :
- souris à jeun
- mesure de la glycémie à T0
- injection intra-péritonéale d'insuline
- mesure de la glycémie 0, 15, 30, 45, 60, 90 min après l'injection
- cinétique réalisée semaines 1 et 12 en prévention.

Le régime HF/HS diminue la sensibilité à l'insuline. TZD rétablit la sensibilité à l'insuline (15 min). Une augmentation significative de la sensibilité à l'insuline est observée chez les souris traitées avec AV102 à 0,1% et 0,25%).

Les différents résultats sont résumés dans le tableau 6 suivant :

**Tableau 6 : impact sur le métabolisme du glucose**

| | **TZD (10 mg/kg/j)** | | **AV102 0,1%** | | **AV102 0,25%)** | |
|---|---|---|---|---|---|---|
| PREVENTION | GLUCOSE | ↓ | GLUCOSE | ↓↓ (3S***) | GLUCOSE | ↓↓ (3S***) |
| | IPGTT | - | IPGTT | - | IPGTT | ↓↓ (12S***) |
| | IPIST | ↓↓ (3S***) | IPIST | - | IPIST | - |
| | INSULINE | ↓ | INSULINE | ↓ | INSULINE | ↓ |
| | **TZD (10 mg/kg/j)** | | **AV102 0,1%** | | **AV102 0,25%)** | |
| INTERVENTION | GLUCOSE | ↓↓ (3S**) | GLUCOSE | - | GLUCOSE | ↓↓ (3S***) |
| | IPGTT | - | IPGTT | - | IPGTT | ↓↓ (6S**) |
| | IPIST | - | IPIST | - | IPIST | - |
| | INSULINE | - | INSULINE | - | INSULINE | - |

| | | | | | | |
|---|---|---|---|---|---|---|
| S=semaine | | | | | | |

En conclusion (impact sur le métabolisme du glucose), l'avocadofurane permet :
de diminuer la glycémie de la souris pré-diabétique ; de pallier la perturbation de la tolérance au glucose induite par le régime HF/HS et de rétablir la sensibilité à l'insuline. L'avocadofurane permet de réguler le métabolisme du glucose de façon plus importante que le TZD chez la souris pré-diabétique et obèse.

### ■ Métabolisme hépatique

Les transaminases (ALAT et ASAT) ne sont pas modifiées par les différents traitements. Une augmentation de l'ALP (phosphatase alcaline) est observée dans tous les groupes. Elle est particulièrement importante dans le groupe AV102 à 0,25%. Il faut noter que dans les groupes contrôles, l'ALP diminue de manière très significative au cours de l'étude, ceci reflétant sans doute la diminution du turn over osseux lié au vieillissement des animaux.

L'augmentation de l'ALP plasmatique dans les groupes TZD et AV102, qui du reste peut-être considérée comme très modeste, ne semble pas être le reflet d'une toxicité hépatique, étant donné que les autres enzymes hépatiques (ALAT et ASAT) ne sont pas modifiées par les différents traitements. Ce résultat serait plus corrélé avec la diminution de la densité osseuse mise en évidence par dexascan.

### 3. Graisses

Groupe prévention : La masse graisseuse corporelle des animaux est significativement diminuée pour AV102 à 314 +/- 72 mg/kg/j (14 et 20 semaines) et à, 136 +/- 24 mg/kg/j (20 semaines). Le TZD est sans effet sur ce paramètre. On observe également une diminution de la leptine pour les deux doses d'AV102.

Groupe intervention : la masse graisseuse corporelle est diminuée après 8 semaines de traitement avec AV102 à 183 +/- 42 mg/kg/j, mais pas à 94 +/- 10 mg/kg/j. On n'observe pas d'effet sur la leptine.

### 4. Autres paramètres

■ Densité osseuse : diminution en prévention (pour les deux doses) à 14 et 20 semaines, ainsi qu'en intervention (AV102 0,25%).
■ Température corporelle : pas de changement pendant la phase de prévention. Légère augmentation en intervention pour TZD et AV102.
■ Dépense énergétiques (calorimétrie) : aucune modification
■ Triglycérides : diminution significative avec AV102 dans le foie et les muscles (prévention; AV102 0,25% et intervention; AV102 0,25%). En prévention, augmentation dans les fèces pour AV102 0,1 %.

### 5. Poids des tissus

Groupe prévention : on observe une diminution significative (>TZD) du poids du tissus adipeux brun avec AV102 (effet dose-dépendent). On n'observe pas de changement du poids du foie en valeur absolue. Toujours en valeur absolue, on observe une diminution de la masse musculaire pour TZD et AV 102 (AV102>TZD).

Groupe intervention : chez les souris traités avec AV102 à 0,25%, on observe une diminution significative du poids du tissus adipeux brun. En valeur absolue, on n'observe pas de changement du poids du foie. Lorsque l'on compare les valeurs absolues de la masse musculaire, on n'observe pas de différence significative entre les différents groupes.

### 6. Histologie.

En prévention et intervention, on observe une diminution de l'accumulation des lipides dans le foie pour les souris traitées avec AV 102 0,25%.

Marquage des mitochondries : on observe une augmentation du marquage pour tous les groupes traités versus HF/HS. L'effet le plus marquant est pour AV102 à 0,25% en intervention.

### 7. Analyse des ARNm

Dans le foie, les expressions ARNm de 8 gènes sont déterminées dans 5 souris de chaque groupe par analyse Q-RT-PCR en temps réel. Les résultats sont donnés dans le tableau 7.
ACO = acetyl coA oxidase = acétyle coA oxydase
PEPCK = phosphoenolpyruvate carboxykinase = carboxykinase phosphoénolpyruvate
GSH-S-transférase = Gluthation-S-transférase
CYP7A1 = Cytochrome P450 7A1
ME= enzyme malique, FAS= synthèse des acides gras, ACC = acétyl-CoA carboxylase

**Tableau 7: analyse des ARNm dans le foie**

| FOIE | TZD | AV102(0,25%) |
|---|---|---|
| ACO (cible PPARα) | ↑ | ↑↑↑ |
| PEPCK (cible PPARγ) | ↑ | ↑↑↑ |
| GSH-S-transférase (cible PXR) | ↑ | ↑ |
| CYP7A1 (conversion oxysterol en acides biliaires) | ↑↑ | ↑↑ |
| ME, FAS, ACC (synthèse acides gras) | ↑↑ | ↑↑ |

AV102 à 0,25% active les agonistes PPARα et PPARγ *in vivo.* AV102 à 0,25%, comme TZD, active également PXR. Enfin, AV102 à 0,25% augmente l'expression des enzymes impliquées dans le métabolisme des lipides et du glucose pour normaliser les effets pathologiques induits par le régime HF/HS.

Dans les tissus adipeux bruns (BAT=Brown adipose tissue) et dans les muscles, on a déterminé l'expression ARNm de 3 gènes dans 5 souris de chaque groupe par analyse Q-RT-PCR en temps réel. Les résultats sont donnés dans le tableau 8.
UCP-1 = Uncoupling protein-1 = protéine-1 non couplée
PGC-1 = co-activateur-1 PPARγ
MCPT-1 = Muscle-type carnitine palmitoyltransférase-1

**Tableau 8: analyse des ARNm dans les muscles et BAT**

| MUSCLE/BAT | TZD | AV102 (0,25%) |
|---|---|---|
| UCP-1 (thermogénèse) | - | - |
| PGC-1 (mitochondrie biogénèse) | ↑ (muscle) | ↑ (muscle) |
| GSH-S-transférase (cible PXR) | ↑ | ↑ |
| MCPT-1 (cible PPARα) entrée des acides gras dans les mitochondries | - | ↑↑ (tissus adipeux) |

AV102 à 0,25% favorise la béta-oxydation dans les BAT. de plus, une augmentation de l'expression de PGC-1 dans les muscles peut contribuer à une augmentation des mitochondries dans les muscles.

### 4) conclusion :

AV102: préviens la prise de poids sous HF/HS, diminue le poids des souris obèses sous régime HF/HS, diminue les TG dans le foie et les muscles, diminue le glucose sanguin et augmente la tolérance au glucose, à des effets sur le cholestérol sont contradictoires mais améliore le bon cholestérol et diminue le mauvais cholestérol, induit l'expression des gènes cibles PPARα et γ dans le foie et le tissus adipeux brun. Dans le muscle tendance à augmenter PGC-1 ce qui pourrait expliquer l'augmentation du marquage des mitochondries dans le muscle. De plus, AV 102 est bien toléré.

En conclusion une diète enrichie en furanne permet de limiter le risque de surpoids ou d'obésité, de diabète de type 2 et de dislipidémie ; ceci devrait se traduire par une baisse concomitante du risque cardiovasculaire.

Chez l'homme, les doses envisageables sont d'environ 60 mg/j d'AV102 ou environ 0,86 mg/kg/j d'AV102 ou environ 2,8.10⁻⁶ mole/kg/j d'AV102.

## Revendications

1. Utilisation d'un ou plusieurs alkyles furannes, synthétiques ou naturels, de formule générale (I) : 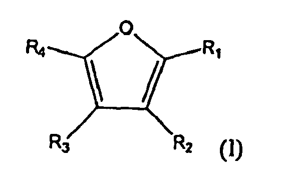 dans laquelle R₁, R₂, R₃ et R₄, identiques ou différents, représentent, indépendamment l'un de l'autre, un atome d'hydrogène, un radical alkyle en C₁-C₃₅, avantageusement en C₁₀-C₂₂, un radical alcényle en C₁-C₃₅, avantageusement en C₁₀-C₂₂, ou un radical alcynyle en C₁-C₃₅, avantageusement en C₁₀-C₂₂, lesdits radicaux alkyles, alcényles et alcynyles pouvant être substitués par un ou plusieurs halogène(s) et/ou par une ou plusieurs fonctions choisies dans le groupe constitué par les fonctions époxyde, hydroxyle (-OH), thiol (-SH), éther (-OR₅), amine primaire (-NH₂), amine secondaire (-NHR₅), amine tertiaire (-NR₅R₆), acétyle (-O-CO-R₅), avec R₅ et R₆ représentant, indépendamment l'un de l'autre, un atome d'hydrogène, un radical alkyle en C₁-C₃₅, avantageusement en C₁₀-C₂₂, ou un radical alcényle en C₁-C₃₅, avantageusement en C₁₀-C₂₂,
pour le traitement cosmétique de la cellulite.

2. Utilisation selon la revendication 1, **caractérisée en ce que** lesdits alkyles furannes sont des 2-alkyles furannes répondant à la formule générale (I) dans laquelle R₁, R₂, R₃ représentent un atome d'hydrogène et R₄ représente un radical alkyle en C₁-C₃₅, avantageusement en C₁₀-C₂₂, un radical alcényle en C₁-C₃₅, avantageusement en C₁₀-C₂₂, ou un radical alcynyle en C₁-C₃₅, avantageusement en C₁₀-C₂₂, lesdits radicaux alkyles, alcényles et alcynyles pouvant être substitués par un ou plusieurs halogène(s) et/ou par une ou plusieurs fonctions choisies dans le groupe constitué par les fonctions époxyde, hydroxyle (-OH), thiol (-SH), éther (-OR₅), amine primaire (-NH₂), amine secondaire (-NHR₅), amine tertiaire (-NR₅R₆), acétyle (-O-CO-R₅), avec R₅ et R₆ représentant, indépendamment l'un de l'autre, un atome d'hydrogène, un radical alkyle en C₁-C₃₅, avantageusement en C₁₀-C₂₂ ou un radical alcényle en C₁-C₃₅, avantageusement en C₁₀-C₂₂.

3. Utilisation selon la revendication 2, **caractérisée en ce que** lesdits alkyles furannes sont des 2-alkyles furannes naturel, notamment présents dans l'insaponifibale d'avocat furanique, répondant à la formule générale (I) dans laquelle R₁, R₂, R₃ représentent un atome d'hydrogène et R₄ représente un radical choisi dans le groupe constitué par les radicaux suivants (*-R₄) :

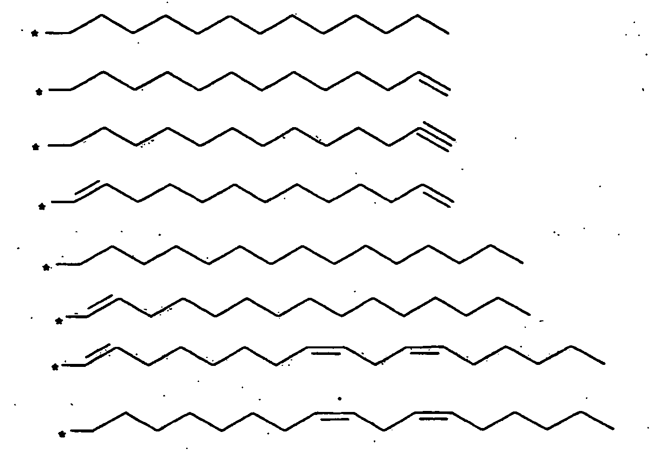

4. Utilisation selon la revendication 3, **caractérisée en ce que** les des 2-alkyles furannes naturels proviennent d'une fraction furanique purifiée de l'insaponifiable d'avocat.

## Claims

1. The use of one or more synthetic or natural alkylfurans of general formula (I): 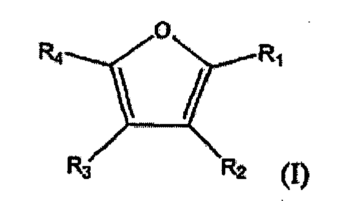 wherein R₁, R₂, R₃ and R₄, either identical or different, represent, independently of each other, a hydrogen, a C₁-C₃₅ alkyl, advantageously a C₁₀-C₂₂ alkyl, a C₁-C₃₅ alkenyl, advantageously a C₁₀-C₂₂ alkenyl, or a C₁-C₃₅ alkynyl, advantageously a C₁₀-C₂₂ alkynyl, wherein said alkyls, alkenyls and alkynyls may be substituted with one or more halogen(s) and/or with one or more functions selected from the group formed by the epoxide, hydroxyl (-OH), thiol (-SH), ether (-OR₅), primary amine (-NH₂), secondary amine (-NHR₅), tertiary amine (-NR₅R₆), acetyl (-O-CO-R₅) functions, with R₅ and R₆ representing, independently of each other, a hydrogen, a C₁-C₃₅ alkyl, advantageously a C₁₀-C₂₂ alkyl, or a C₁-C₃₅ alkenyl, advantageously a C₁₀-C₂₂ alkenyl, for cosmetically treating cellulitis.

2. The use according to claim 1, **characterized in that** said alkylfurans are 2-alkylfurans described by general formula (I) wherein R₁, R₂ and R₃ represent a hydrogen and R₄ represents a C₁-C₃₅ alkyl, advantageously a C₁₀-C₂₂ alkyl, a C₁-C₃₅ alkenyl, advantageously a C₁₀-C₂₂ alkenyl, or a C₁-C₃₅ alkynyl, advantageously a C₁₀-C₂₂ alkynyl, said alkyls, alkenyls and alkynyls may be substituted with one or more halogen(s) and/or with one or more functions selected from the group formed by the epoxide, hydroxyl (-OH), thiol (-SH), ether (-OR₅), primary amine (-NH₂), secondary amine (-NHR₅), tertiary amine (-NR₅R₆), acetyl (-O-CO-R₅) functions, with R₅ and R₆ representing, independently of each other, a hydrogen, a C₁-C₃₅ alkyl, advantageously a C₁₀-C₂₂ alkyl, or a C₁-C₃₅ alkenyl, advantageously a C₁₀-C₂₂ alkenyl.

3. The use according to claim 2, **characterized in that** said alkylfurans are natural 2-alkylfurans, notably present in furanic unsaponifiable of avocado, described by general formula (I) wherein R₁, R₂, R₃ represent a hydrogen and R₄ represents a radical selected from the group formed by the following radicals (*-R₄):

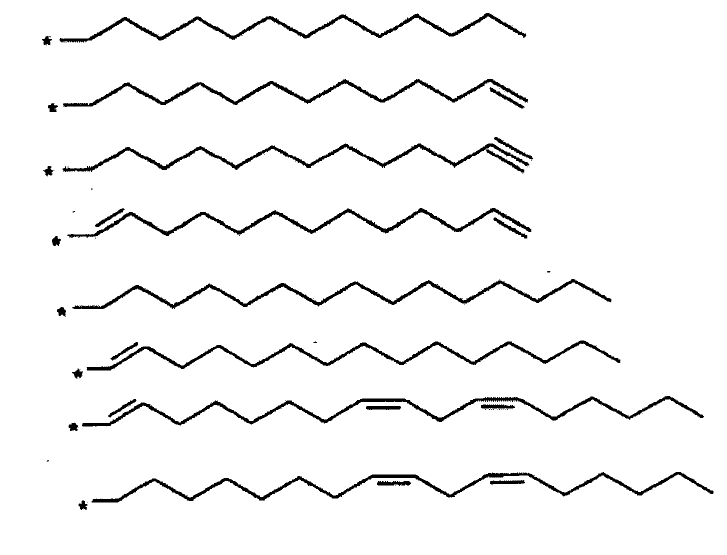

4. The use according to claim 3, **characterized in that** said natural 2-alkylfurans derive from a purified furanic fraction from unsaponifiable of avocado.

## Patentansprüche

1. Verwendung eines oder mehrerer synthetischer oder natürlicher Alkylfurane der allgemeinen Formel (I): 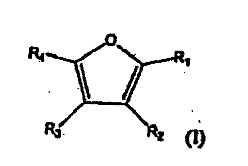 in der R₁, R₂, R₃ und R₄, identisch oder verschieden, unabhängig voneinander ein Wasserstoffatom, einen (C₁-C₃₅)-, vorteilhaft (C₁₀-C₂₂)-Alkylrest, einen (C₁-C₃₅)-, vorteilhaft (C₁₀-C₂₂)-Alkenylrest oder einen (C₁-C₃₅)-, vorteilhaft (C₁₀-C₂₂)-Alkinylrest darstellen, wobei die Alkyl-, Alkenyl- und Alkinylreste mit einem oder mehreren Halogen(en) und/oder mit einer oder mehreren Funktionen substituiert sein können, welche aus der Gruppe ausgewählt sind, die aus Epoxid-, Hydroxyl(-OH)-, Thiol(-SH)-, Ether(-OR₅)-, primären Amin(-NH₂)-, sekundären Amin(-NHR₅)-, tertiären Amin(-NR₅R₆)-, Acetyl(-O-CO-R₅)-Funktionen besteht, wobei R₅ und R₆ unabhängig voneinander ein Wasserstoffatom, einen (C₁-C₃₅)-, vorteilhaft (C₁₀-C₂₂)-Alkylrest oder einen (C₁-C₃₅)-, vorteilhaft (C₁₀-C₂₂)-Alkenylrest darstellen, für die kosmetische Behandlung von Cellulite.

2. Verwendung nach Anspruch 1, **dadurch gekennzeichnet, dass** die Alkylfurane 2-Alkylfurane sind, welche der allgemeinen Formel (I) entsprechen, in der R₁, R₂, R₃ ein Wasserstoffatom darstellen und R₄ einen (C₁-C₃₅)-, vorteilhaft (C₁₀-C₂₂)-Alkylrest, einen (C₁-C₃₅)-, vorteilhaft (C₁₀-C₂₂)-Alkenylrest oder einen (C₁-C₃₅)-, vorteilhaft (C₁₀-C₂₂)-Alkinylrest darstellen, wobei die Alkyl-, Alkenyl- und Alkinylreste mit einem oder mehreren Halogen(en) und/oder mit einer oder mehreren Funktionen substituiert sein können, die ausgewählt sind aus der Gruppe bestehend aus Epoxid-, Hydroxyl(-OH)-, Thiol(-SH)-; Ether(OR₅)-, primären Amin(-NH₂)-, sekundären Amin(-NHR₅)-, tertiären Amin(-NR₅R₆)-, Acetyl(-O-CO-R₅)-Funktionen, wobei R₅ und R₆ unabhängig voneinander ein Wasserstoffatom, einen (C₁-C₃₅)-, vorteilhaft (C₁₀-C₂₂)-Alkylrest oder einen (C₁-C₃₅)-, vorteilhaft (C₁₀-C₂₂)-Alkenylrest darstellen.

3. Verwendung nach Anspruch 2, **dadurch gekennzeichnet, dass** die Alkylfurane natürliche 2-Alkylfurane sind, die insbesondere in dem Furan-haltigen unverseifbaren Teil von Avocado vorliegen und der allgemeinen Formel (I) entsprechen, in der R₁, R₂, R₃ ein Wasserstoffatom darstellen und R₄ einen Rest darstellt, der ausgewählt ist aus der Gruppe bestehend aus den folgenden Resten (*-R₄):

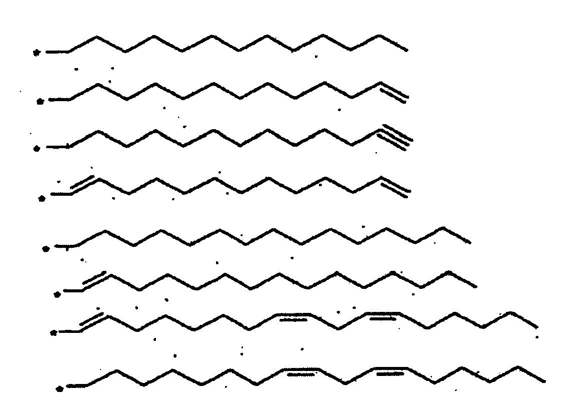

4. Verwendung nach Anspruch 3, **dadurch gekennzeichnet, dass** die natürlichen 2-Alkylfurane aus einer Furan-haltigen Fraktion stammen, die aus dem unverseifbaren Teil von Avocado gereinigt wurde.
